(19) Europäisches Patentamt
European Patent Office
Office européen des brevets

(11) EP 2 749 294 A1

(12) **EUROPEAN PATENT APPLICATION**

(43) Date of publication:
**02.07.2014 Bulletin 2014/27**

(21) Application number: **13199364.4**

(22) Date of filing: **23.12.2013**

(51) Int Cl.:
*A61K 51/04* (2006.01)
*A61K 31/555* (2006.01)
*A61K 31/513* (2006.01)
*A61K 31/519* (2006.01)
*A61K 31/52* (2006.01)
*A61P 35/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.12.2012 JP 2012289452**
**04.12.2013 JP 2013250743**

(71) Applicants:
- **NIHON MEDI-PHYSICS CO., LTD.**
  **Tokyo 136-0075 (JP)**
- **National Institute of Radiological Sciences**
  **Chiba-shi,**
  **Chiba 263-8555 (JP)**
- **National Cancer Center**
  **Tokyo 104-0045 (JP)**

(72) Inventors:
- **Yoshii, Yukie**
  **Chiba-shi,, Chiba 263-8555 (JP)**
- **Furukawa, Takako**
  **Chiba-shi,, Chiba 263-8555 (JP)**
- **Saga, Tsuneo**
  **Chiba-shi,, Chiba 263-8555 (JP)**
- **Matsumoto, Hiroki**
  **Sodegaura-shi,, Chiba (JP)**
- **Yoshimoto, Mitsuyoshi**
  **Chuo-ku,, Tokyo 104-0045 (JP)**

(74) Representative: **TBK**
**Bavariaring 4-6**
**80336 München (DE)**

# (54) Anti-tumor agent and anti-tumor kit

(57) An anti-tumor agent comprising a radioactive diacetyl-bis(N4-methylthiosemicarbazone) copper complex (Cu-ATSM), wherein the anti-tumor agent is used for combined administration with a metabolic inhibitor, in particular 5-fluorouracil, tegafur, capecitabine, 6-thioguanine or pemetrexed.



Printed by Jouve, 75001 PARIS (FR)

## Description

This application is based on Japanese patent application No. 2012-289452 and 2013-250743, the content of which are incorporated hereinto by reference.

## BACKGROUND

## TECHNICAL FIELD

The present invention relates to an anti-tumor agent and an anti-tumor kit.

## RELATED ART

A radioactive dithiosemicarbazone copper complex is known as a diagnostic agent for hypoxic sites or mitochondrial dysfunction (for example, Japanese Patent Laid-Open No. H08-245425). Jason S. Lewis et al. (2001), Pros. Natl. Acad. Sci. vol. 98, 1206-1211 also discloses that a radioactive diacetyl-bis(N4-methylthiosemicarbazone) copper complex (hereinafter also referred to as "radioactive Cu-ATSM") is useful as a radiotherapeutic agent for tumor targeting hypoxic regions.

In recent years, it has also been revealed that $^{64}$Cu-ATSM accumulates in CD133-positive cells (Yukie Yoshii et al. (2010), Nucl. Med. Biol. vol. 37, 395-404). Yukie Yoshii et al. (2011), Nucl. Med. Biol. vol. 38, 151-157 reports that it decreased the amount of CD133-positive cells in tumor to shrink the tumor. Thus, the radioactive Cu-ATSM is also expected to be useful as an agent for detecting cancer stem cells and as a preventive/therapeutic agent for cancer targeting cancer stem cells (Japanese Patent Laid-Open No. 2010-13380).

## SUMMARY

However, according to the disclosure of Japanese Patent Laid-Open No. 2010-13380, $^{64}$Cu-ATSM is shown to accumulate in sites different from those for $^{18}$FDG in tumor. Thus, the administration of the radioactive Cu-ATSM alone has a problem that it cannot effectively kill tumor regions in which the radioactive Cu-ATSM does not accumulate.

If the dose of the radioactive Cu-ATSM is increased, even the tumor regions in which the radioactive Cu-ATSM does not accumulate may be able to be killed by radioactive rays emitted by the radioactive Cu-ATSM. However, there is concern that the exposure dose of radiation on normal tissue is increased.

Made in view of the above-described circumstances, the present invention provides a technology to increase the anti-tumor effect of a radioactive Cu-ATSM.

The present inventors have newly found that the combined use of a radioactive Cu-ATSM and a metabolic inhibitor provides a synergistic killing effect against tumor cells. It has also been found that the combined administration of a radioactive Cu-ATSM and a metabolic inhibitor increases a killing effect against cancer stem cells compared to the administration of the radioactive Cu-ATSM alone.

One aspect of the present invention provides an anti-tumor agent comprising a radioactive diacetyl-bis(N4-methylthiosemicarbazone) copper complex (a radioactive Cu-ATSM), wherein the anti-tumor agent is used for combined administration with a metabolic inhibitor.

Another aspect of the present invention provides use of a metabolic inhibitor for combined administration with a radioactive Cu-ATSM.

In addition, another aspect of the present invention provides an anti-tumor kit comprising a metabolic inhibitor and a radioactive Cu-ATSM.

According to the present invention, a high killing effect can be obtained against cancer stem cells and tumor cells, and the anti-tumor effect of a radioactive Cu-ATSM can be increased.

## BRIEF DESCRIPTION OF THE DRAWINGS

The above and other objects, advantages and features of the present invention will be more apparent from the following description of certain preferred embodiments taken in conjunction with the accompanying drawing.

FIG. 1 is a graph showing changes with time in the tumor volume in Examples and Comparative Examples;
FIG. 2 is a graph showing the percentage of apoptotic cells in tumor in Examples and Comparative Examples;
FIG. 3 is a graph showing the percentage of CD133-positive cells in tumor in Examples and Comparative Examples;
FIG. 4 is a graph showing changes with time in the tumor volume in Examples and Comparative Examples; and
FIG. 5 is a graph showing changes with time in the tumor volume in Examples and Comparative Examples.

DETAILED DESCRIPTION

The invention will be now described herein with reference to illustrative embodiments. Those skilled in the art will recognize that many alternative embodiments can be accomplished using the teachings of the present invention and that the invention is not limited to the embodiments illustrated for explanatory purposed.

[Anti-tumor Agent]

The anti-tumor agent of the present invention comprises a radioactive Cu-ATSM represented by formula (1) below.

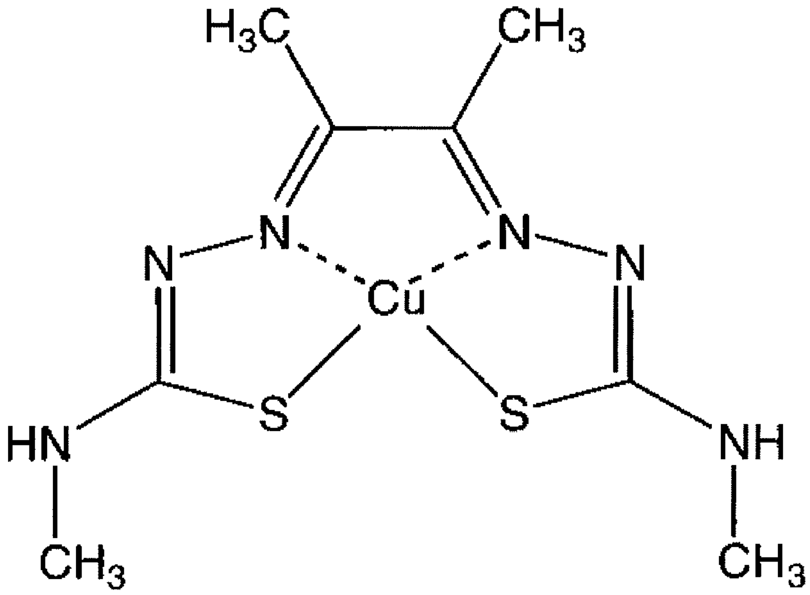

(1)

In the formula (1), Cu represents a radioactive isotope; however, in view of being capable of more effectively killing tumor cells, it is preferably $^{64}Cu$ or $^{67}Cu$ because of emitting beta rays.

The radioactive Cu-ATSM can be produced, for example, by the method of Jalilian et al. (Acta Pharmaceutica, 59(1), 2009, pp. 45-55), a method as described in "PET yo Houshaseiyakuzai no Seizo oyobi Hinshitsukanri - Gousei to Rinshoushiyou eno Tebiki (Production and Quality Control of Radiopharmaceuticals for PET - A Handbook for Synthesis and Clinical Use)" (edited by PET Kagaku Workshop (PET Chemistry Workshop)) Fourth Edition (Revised in 2011), the method of Tanaka et al. (Nuclear Medicine and Biology, vol. 33, 2006, pp. 743-50), or the method of Lewis et al. (J. Nucl. Med., 2001, 42, 655-661).

The radioactive Cu-ATSM can accumulate in various tumors. Examples of the tumors in which the radioactive Cu-ATSM accumulates include breast cancer, brain tumor, prostate cancer, pancreas cancer, stomach cancer, lung cancer, colon cancer, rectal cancer, large bowel cancer, small intestinal cancer, esophageal cancer, duodenal cancer, tongue cancer, pharyngeal cancer, salivary gland cancer, schwannoma, liver cancer, kidney cancer, bile duct cancer, endometrium cancer, uterocervical cancer, ovary cancer, bladder cancer, skin cancer, angioma, malignant lymphoma, malignant melanoma, thyroid cancer, parathyroid cancer, nasal cancer, paranasal cancer, bone tumor, angiofibroma, retinosarcoma, penis cancer, testis tumor, pediatric solid cancer, sarcoma, and leukemia. These tumors may be primary or metastatic.

For the purpose of the present invention, "anti-tumor" refers to suppressing the increase of tumor and, further, decreasing or extinguishing tumor, and the "anti-tumor agent" of the present invention refers to an agent containing, as an active ingredient, an ingredient capable of killing tumor cells together with cancer stem cells, suppressing the proliferation of these tumors and, further, decreasing or extinguishing tumor.

The anti-tumor agent of the present invention may directly comprise the radioactive Cu-ATSM as an active ingredient, or may be obtained by formulating it together with a pharmacologically acceptable carrier, diluent, or excipient. The dosage form may be for oral administration or for parenteral administration; however, a dosage form for parenteral administration such as, for example, injection is preferable.

The anti-tumor agent of the present invention is used for combined administration with a metabolic inhibitor to be described later. For the purpose of the present invention, "combined administration" refers to being used in the same administration regimen, and the agents need only to be administered so that the same therapeutic effect (the effect of

suppressing the proliferation of tumor, and, further, shrinking or extinguishing it) can be exerted on the same tumor region. The metabolic inhibitor may be administered before the administration of the anti-tumor agent; the metabolic inhibitor may be administered after the administration of the anti-tumor agent; or the anti-tumor agent and the metabolic inhibitor may be simultaneously administered.

The anti-tumor agent of the present invention may preferably be administered after the administration of the metabolic inhibitor, and the anti-tumor agent of the present invention may more preferably be administered after the single administration (preferably, sustained administration) or repeated administration of the metabolic inhibitor. For the purpose of the present invention, "repeated administration" refers to administrating a plurality of times at fixed time intervals, and, for example, the administration may be performed once or a plurality of times a day for 2 days to 1 month. "Sustained administration" refers to continuous administration for a fixed time, and, for example, the administration may be continuously performed for several hours to 1 week.

When the anti-tumor agent of the present invention is administered after the administration of the metabolic inhibitor, the administration interval between the metabolic inhibitor and the anti-tumor agent is not particularly limited; however, for example, the anti-tumor agent of the present invention may be administered after the blood concentration of the active ingredient (for example, 5-fluorouracil, 6-thioguanine, or pemetrexed) of the metabolic inhibitor in the body has reached the maximum blood concentration.

A subject to which the anti-tumor agent of the present invention is to be administered is, for example, a mammal, preferably a human. The dose of the anti-tumor agent of the present invention vary depending on the type, age, sex, body weight, and symptoms of a patient to which it is to be administered, the method for administration, and the like, and are not particularly limited; however, the range thereof may be adopted which is generally adopted for typical radiopharmaceuticals. The anti-tumor agent of the present invention may be administered singly or may be administered a plurality of times.

The anti-tumor agent comprising a radioactive Cu-ATSM can be used in combination with the metabolic inhibitor to increase the cancer stem cell-killing effect of the radioactive Cu-ATSM. Thus, the anti-tumor agent of the present invention can also be used as a cancer stem cell-killing agent for killing cancer stem cells. Whether cancer stem cells have been killed or not can be confirmed, for example, by measuring the expression of a cell surface marker expressed by the cancer stem cells using flow cytometry or by preparing sections of tumor and immunohistostaining them using an antibody capable of specifically binding to cancer stem cells. The cell surface marker expressed by cancer stem cells is known to vary depending on the type of the tumor; there are CD34+ and the like for acute myelocytic leukemia (AML); CD133+ and the like for brain tumor, medulloblastoma, glioblastoma, ependymoma, large bowel cancer, breast cancer, and malignant melanoma; CD44+ and the like for pancreas cancer, breast cancer, and prostate cancer; and CD20+ and the like for melanoma.

[Metabolic Inhibitor]

For the purpose of the present invention, the metabolic inhibitor is a compound similar in chemical structure to a substance serving as a material to make up nucleic acid in the division/proliferation of cancer cells or an agent containing it as an active ingredient, which compound is an anti-cancer agent that interferes with the biosynthesis of nucleic acids or the biosynthetic pathways for nucleic acids to suppress proliferation. Preferably, the metabolic inhibitor may be one or more agents selected from the group consisting of pyrimidine metabolism antagonists, purine metabolism antagonists, and folate metabolism antagonists.

The pyrimidine metabolism antagonist is preferably a thymidylate syntase inhibitor. The thymidylate syntase inhibitor is an agent causing impaired synthesis of DNA by inhibiting thymidylate synthase; examples thereof include fluorinated pyrimidine agents. The fluorinated pyrimidine agent may be any agent that provides the body with 5-fluorouracil upon its administration, and may be one containing 5-fluorouracil or a prodrug thereof as an active ingredient. As the fluorinated pyrimidine agent, an agent comprising one or more compounds selected from the group consisting of 5-fluorouracil, tegafur, capecitabine, and salts thereof as an active ingredient may be preferably adopted. When 5-fluorouracil, tegafur, or capecitabine forms a salt, the salt may be any salt that is pharmacologically acceptable.

The fluorinated pyrimidine agents may be various commercially available ones. Examples of 5-fluorouracil include 5-FU (trade name). Examples of tegafur include Futraful (R). According to the present invention, the fluorinated pyrimidine agent may be a mixture such as tegafur/uracil (UFT (R)) or tegafur/gimeracil/oteracil potassium (TS-1 (R)).

Examples of the purine metabolism antagonist include one containing one or more compounds selected from the group consisting of 6-mercaptopurine, azathioprine, 6-thioguanine, and salts thereof as active ingredients. When 6-mercaptopurine, azathioprine, or 6-thioguanine forms a salt, the salt may be any salt that is pharmacologically acceptable. They may be various commercially available ones. Examples of the commercially available 6-mercaptopurine include Purinethol and Leukerin. Examples of the commercially available azathioprine include Azanin and Imuran.

Examples of the folate metabolism antagonist include one containing one or more compounds selected from the group consisting of methotrexate, pemetrexed, and salts thereof as active ingredients. When methotrexate or pemetrexed forms a salt, the salt may be any salt that is pharmacologically acceptable. They may be various commercially available ones; examples of the commercially available methotrexate include Methotrexate. Examples of the commercially available pemetrexed include Alimta (R).

According to the present invention, the metabolic inhibitor may be obtained by formulating an active ingredient directly or after properly adding a pharmacologically acceptable carrier, excipient, binder, lubricant, disintegrator, sustained-release agent, buffer, coating agent, colorant, or the like to the active ingredient, and may be have a dosage form suitable for oral administration or parenteral administration. Examples thereof include oral agents such as tablets, capsules, powders, granules, and syrups and parenteral agents such as injections, external preparations, suppositories, pellets, drops, and sustained release preparations. Two or more types of dosage forms may be combined.

The metabolic inhibitor may be any of those that can be used for combined administration with the radioactive Cu-ATSM, and may be singly administered (preferably, sustainedly administered) or repeatedly administered before or after the administration of the anti-tumor agent of the present invention. It may be administered both before and after the administration of the anti-tumor agent of the present invention or may be administered simultaneously with the anti-tumor agent; however, it is preferably administered before the administration of the anti-tumor agent of the present invention.

The dose of the metabolic inhibitor administered in combination with the anti-tumor agent of the present invention is not particularly limited; however, the metabolic inhibitor can preferably be used in a range that does not exceed the dose of the metabolic inhibitor when used alone as an anti-tumor drug, and the metabolic inhibitor is more preferably administered in such a dose that no body weight reduction is observed when administered alone. Specifically, the dose can be set so that the rate of body weight reduction after the administration of the metabolic inhibitor is 10% or less, preferably 9% or less, more preferably 8% or less, still more preferably 7% or less when the metabolic inhibitor is administered alone. This can increase the anti-tumor effect of the radioactive Cu-ATSM while reducing side effects of the metabolic inhibitor. The administration alone as used here refers to an administration regimen in which another pharmaceutical containing the radioactive Cu-ATSM is not used in combination. The rate of body weight reduction can be expressed by "Body weight reduction rate (%) = {(Body weight before starting administration of metabolic inhibitor - Body weight after starting administration of metabolic inhibitor)/Body weight before starting administration of metabolic inhibitor} x 100"; the "body weight after starting administration of metabolic inhibitor" may be preferably body weight at 1 to 2 weeks after starting the administration of the metabolic inhibitor.

The metabolic inhibitor can be used in combination with the anti-tumor agent comprising a radioactive Cu-ATSM to kill cancer stem cells. Thus, the metabolic inhibitor used in combined administration with the anti-tumor agent of the present invention can be a cancer stem cell-killing agent.

[Anti-tumor Kit]

The anti-tumor kit of the present invention comprises the anti-tumor agent comprising a radioactive Cu-ATSM and the metabolic inhibitor.

The anti-tumor kit of the present invention preferably comprises a package insert informing that the anti-tumor agent is administered after the administration of the metabolic inhibitor. The package insert may describe a method for using the anti-tumor kit of the present invention to be described later.

The anti-tumor kit of the present invention is preferably used by administering the anti-tumor agent and the metabolic inhibitor to a mammal. The mammal to which they are to be administered is more preferably a human.

[Method for Using Anti-tumor Agent, Metabolic Inhibitor, and Anti-tumor Kit]

An example of a method for using each of the anti-tumor agent, the metabolic inhibitor, and the anti-tumor kit of the present invention will be described below.

First, the metabolic inhibitor is administered to a test subject or a patient developing tumor. Here, the dose of the metabolic inhibitor can be set so that the rate of body weight reduction after the administration of the metabolic inhibitor is 10% or less, preferably 9% or less, more preferably 8% or less, still more preferably 7% or less when the metabolic inhibitor is administered alone.

Thereafter, the anti-tumor agent comprising a radioactive Cu-ATSM is administered. The anti-tumor agent is preferably administered after the blood concentration of the active ingredient (for example, 5-fluorouracil, 6-thioguanine, or pemetrexed) of the metabolic inhibitor in the body has reached the maximum blood concentration after the administration of the metabolic inhibitor.

The dosage schedule consisting of the repeated administration of the metabolic inhibitor and the single administration of the anti-tumor agent comprising a radioactive Cu-ATSM may be repeated a plurality of times. This repeat may be continuously performed, or may be performed at 1- to 30-day intervals, preferably 10- to 25-day intervals after the administration of the anti-tumor agent.

This allows the radioactive Cu-ATSM and the metabolic inhibitor to act synergistically to kill tumor cells including cancer stem cells. Thus, the anti-tumor kit of the present invention can also be used as a cancer stem cell-killing agent for killing cancer stem cells. The anti-tumor kit of the present invention can provide a high tumor proliferation-suppressing effect because it combines a tumor cell-killing effect and a radiosensitizing effect. It also has a high anti-tumor effect with respect to the amount of administered radioactivity; thus, the exposure of normal tissue to radiation can be suppressed. The dose of the metabolic inhibitor is low to such an extent that the body weight is not decreased; thus, side effects of the metabolic inhibitor can also be decreased.

Embodiments of the present invention have been described above. However, the present invention is not limited to the embodiments and various modifications can be made. For example, the present invention includes the following technical idea.

[1] An anti-tumor agent comprising a radioactive diacetyl-bis(N4-methylthiosemicarbazone) copper complex, wherein the anti-tumor agent is used for combined administration with a fluorinated pyrimidine agent.
[2] The anti-tumor agent according to [1], wherein the fluorinated pyrimidine agent contains one or more compounds selected from the group consisting of 5-fluorouracil, tegafur, capecitabine, and salts thereof as an active ingredient.
[3] The anti-tumor agent according to [1] or [2], wherein the radioactive diacetyl-bis(N4-methylthiosemicarbazone) copper complex is $^{64}$Cu-ATSM or $^{67}$Cu-ATSM.
[4] The anti-tumor agent according to any one of [1] to [3], wherein the anti-tumor agent is used as a cancer stem cell-killing agent.
[5] The anti-tumor agent according to any one of [1] to [4], wherein the anti-tumor agent is used after the administration of the fluorinated pyrimidine agent.
[6] The anti-tumor agent according to any one of [1] to [5], wherein the dose of the fluorinated pyrimidine agent is set so that when the fluorinated pyrimidine agent is administered alone, the rate of body weight reduction after the administration of the fluorinated pyrimidine agent is 10% or less.
[7] Use of a fluorinated pyrimidine agent for combined administration with a radioactive diacetyl-bis(N4-methylthiosemicarbazone) copper complex.
[8] Use of the fluorinated pyrimidine agent according to [7], wherein the fluorinated pyrimidine agent is used as a cancer stem cell-killing agent.
[9] An anti-tumor kit comprising a fluorinated pyrimidine agent and a radioactive diacetyl-bis(N4-methylthiosemicarbazone) copper complex.
[10] The anti-tumor kit according to [9], wherein the anti-tumor kit is used for killing cancer stem cells.

EXAMPLES

The present invention will be described in further detail by describing Examples. However, the present invention is not intended to be limited to the contents thereof.

(Production Example 1) Preparation of $^{64}$Cu-ATSM and Metabolic Inhibitor

(Synthesis of ATSM)

The synthesis of diacetyl-bis(N4-methylthiosemicarbazone) (ATSM) was performed according to the method of Tanaka et al. (Nuclear Medicine and Biology, vol. 33, 2006, pp. 743-50).

(Synthesis of $^{64}$Cu-ATSM)

$^{64}$Cu was produced and purified according to the method of McCarthy et al. (Nuclear medicine and biology, vol. 24, 1997, pp. 35-43) and the method of Obata et al. (Nuclear medicine and biology, vol. 30, 2003, pp. 535-539). $^{64}$Cu-ATSM was synthesized according to the method of Tanaka et al. (supra) by using ATSM and $^{64}$Cu. The produced agent was tested using a thin layer chromatography method (TLC method), and one having a radiochemical purity of 95% or more was used for the following experiment. Conditions of analysis of $^{64}$Cu-ATSM using TLC are as follows.

TLC plate: silica gel plate (product name: Silica gel 60, from Merck Ltd. Japan)
Development phase: ethyl acetate
Detection: fluoroimage analyzer (Model: FLA-7000, from Fujifilm Corporation)

(Preparation of 5-Fluorouracil Solution)

5-Fluorouracil (from Wako) was dissolved in saline, which was adjusted to 100 μL/mouse and used for the following experiment. The 5-fluorouracil solution is abbreviated as "5-FU" in the following Examples and corresponding drawings.

(Preparation of 6-Thioguanine solution)

6-Thioguanine (from Wako) was dissolved in saline, which was adjusted to 100 μL/mouse and used for the following experiment. The 6-thioguanine solution is abbreviated as "6TG" in the following Examples and corresponding drawings.

(Preparation of Pemetrexed Solution)

Pemetrexed (Alimta (R) Injection, from Eli Lilly Japan K. K.) was dissolved in saline, which was adjusted to 100 μL/mouse and used for the following experiment. The pemetrexed solution is abbreviated as "PT" in the following Examples and corresponding drawings.

(Example 1) Study of Dose of 5-FU Using Nude Mouse

5-FU was repeatedly intraperitoneally administered to BALB/c nude mice (male, 7-week old, about 25 g in body weight, obtained from Japan SLC, Inc.) at 25 mg/kg, 50 mg/kg, or 100 mg/kg once a day for 4 days. Body weight was measured at the 5th day from the first starting day of administration and later to determine the rate of body weight reduction. The rate of body weight reduction was calculated based on the following equation.

Body weight reduction rate (%) = [{(Body weight at day prior to the date of first 5-FU administration) − (Body weight measured after start of 5-FU administration)}/(Body weight at day prior to the date of first 5-FU administration)] × 100

Saline was administered to a control group in place of 5-FU. The results are shown in Table 1. In Table 1, they were expressed in average and standard deviation for 4 mice. Mice receiving the administration of 50 mg/kg of 5-FU died at the 10th or 11th day from administration, and mice receiving the administration of 100 mg/kg of 5-FU died at the 7th or 8th day from administration; thus, there are no measured data of body weight after death.

Table 1

| | | Elapsed Days from First Start of Administration | | | | |
|---|---|---|---|---|---|---|
| | | 5th Day | 9th Day | 12th Day | 16th Day | 18th Day |
| Rate of Body Weight Reduction (%) | Control Group | 0.46±3.71 | 2.61±4.45 | 0.63±6.31 | 2.30±7.86 | 1.05±7.23 |
| | 25mg/kg/day | 6.56±0.78 | 3.55±1.28 | 4.73±2.29 | 1.10±1.97 | -1.33±2.81 |
| | 50mg/kg/day | 16.09±1.54 | 29.38±3.22 | - | - | - |
| | 100mg/kg/day | 22.59±1.44 | - | - | - | - |

As shown in Table 1, the rate of body weight reduction exceeded 10% at the 5th day in the group receiving the administration of 50 mg/kg or 100 mg/kg per day while the rate of body weight reduction was within 10% even at the 18th day in the group receiving the administration of 25 mg/kg per day. Accordingly, in subsequent Examples, 5-FU was decided to be repeatedly administered at 25 mg/kg once a day for 4 days.

(Example 2) Anti-tumor Effect of Administration of $^{64}$Cu-ATSM and 5-FU in HT29 Tumor-Bearing Mouse

Human large bowel cancer-derived HT29 cells were purchased from ATCC and proliferated for use. The HT29 tumor-bearing model was prepared by implanting 1 x $10^7$ HT29 cells subcutaneously in the femoral region of BALB/c nude mice (male, 7-week old, about 25 g in body weight, obtained from Japan SLC, Inc.), and 1 week after implantation, 5-FU was repeatedly intraperitoneally administered at 25 mg/kg once a day for 4 days. At the final day of 5-FU administration, 37 MBq (1 mCi) of $^{64}$Cu-ATSM was administered through the tail vein. Saline was administered to a control group in place of 5-FU and $^{64}$Cu-ATSM. The tumor size in mice was measured 2, 4, 8, 12, or 15 days after starting the administration of 5-FU. Cells obtained from the tumor removed at 2 days after the administration of $^{64}$Cu-ATSM were subjected to the detection of apoptotic cells and CD133-positive (CD133+) cells using flow cytometry (Guava flow cytometry, from Millipore Corporation). The detection of apoptotic cells was performed using Annexin V (Mouse Anti-Human Annexin V FITC, AbD MCA2712F, from Serotec Co., Ltd.). The detection of CD133+ cells was performed using an anti-CD133 antibody (CD133/1(A133)-PE, from Miltenyi Biotec K.K.).

(Comparative Example 1) Anti-Tumor Effect of Administration of 5-FU Alone in HT29 tumor-Bearing Mouse

The experiment was carried out in the same way as Example 2 except for administering saline in place of $^{64}$Cu-ATSM.

(Comparative Example 2) Anti-Tumor Effect of Administration of $^{64}$Cu-ATSM Alone in HT29 tumor-Bearing Mouse

The experiment was carried out in the same way as Example 2 except for administering saline in place of 5-FU.

(Comparative Example 3) Anti-Tumor Effect of Administration of $^{64}$Cu-ATSM Alone in HT29 tumor-Bearing Mouse

Saline was administered in place of 5-FU. The dose of $^{64}$Cu-ATSM was 74 MBq (2 mCi). The experiment was carried out in the otherwise same way as Example 2.

[Evaluation 1]

The volume of tumor was calculated from the size of tumor measured in Example 2 and Comparative Examples 1 to 3; the change thereof with time is shown in FIG. 1. In FIG. 1, the tumor volume at the day prior to the day of the start of 5-FU administration was set to 100%, and the percentage based thereon was given. In FIG. 1, data are expressed in average and standard deviation for 6 mice. As shown in FIG. 1, the administration of 5-FU alone did not suppress the proliferation of tumor. The administration of $^{64}$Cu-ATSM alone suppressed the proliferation of tumor depending on the dose thereof, while the combined administration of 37 MBq of $^{64}$Cu-ATSM and 5-FU had a higher tumor proliferation-suppressing effect than the administration of 74 MBq of $^{64}$Cu-ATSM alone (Comparative Example 3).

FIG. 2 shows the results of detecting apoptotic cells in Example 2 and Comparative Examples 1 to 3. In FIG. 2, the results of percentages of apoptotic cells in the removed tumor are expressed in average and standard deviation for 6 mice. As shown in FIG. 2, the apoptosis of tumor cells was confirmed in each of the single administrations of 5-FU or $^{64}$Cu-ATSM alone while the combined use of $^{64}$Cu-ATSM and 5-FU resulted in the detection of apoptotic cells in an amount exceeding the cumulative amount of apoptotic cells resulting from both of the single administrations.

FIG. 3 shows the results of detecting CD133+ cells in Example 2 and Comparative Examples 1 to 3. In FIG. 3, the results of percentages of CD133+ cells in the removed tumor are expressed in average and standard deviation for 6 mice. FIG. 3 showed that even single administrations of 5-FU or $^{64}$Cu-ATSM alone can kill CD133+ cells while the combined administration of $^{64}$Cu-ATSM and 5-FU can kill more CD133+ cells.

The above results showed that the combined use of $^{64}$Cu-ATSM and 5-FU in an amount causing no body weight reduction was noted to have a significant anti-tumor effect without the observation of body weight reduction.

(Example 3) Study of Dose of 6TG Using Nude Mouse

6TG was repeatedly intraperitoneally administered to BALB/c nude mice (male, 7-week old, about 25 g in body weight, obtained from Japan SLC, Inc.) at 12.5 mg/kg, 25 mg/kg, or 50 mg/kg once a day for 4 days. Body weight was measured at the 5th day from the first starting day of administration and later to determine the rate of body weight reduction. The rate of body weight reduction was calculated based on the following equation.

Body weight reduction rate (%) = [{(Body weight at day prior to the date of first 6TG administration) − (Body weight measured after start of 6TG administration)}/(Body weight at day prior to the date of first 6TG administration)] × 100

Saline was administered to a control group in place of 6TG. The results are shown in Table 2. In Table 2, they were expressed in average and standard deviation for 4 mice. Mice receiving the administration of 25 mg/kg of 6TG died at the 13th to 15th day from administration, and mice receiving the administration of 50 mg/kg of 6TG died at the 6th to 8th day from administration; thus, there are no measured data of body weight after death.

**Table 2**

| | | Elapsed Days from First Start of Administration | | | | |
|---|---|---|---|---|---|---|
| | | 5th Day | 9th Day | 12th Day | 16th Day | 18th Day |
| Rate of Body Weight Reduction (%) | Control Group | - 5.48±1.80 | -9.03±6.04 | -12.1±8.73 | -18.89±5.63 | -12.57±5.61 |
| | 12.5mg/kg/day | 6.79±7.85 | -1.48±5.75 | -2.99±7.67 | -5.86±7.4 | -7.22±7.22 |
| | 25mg/kg/day | 11.93±8.40 | 20.58± 15.29 | 19.84±19.68 | - | - |
| | 50mg/kg/day | 16.75±0.57 | - | - | - | - |

As shown in Table 2, the rate of body weight reduction exceeded 10% at the 5th day in the group receiving the administration of 25 mg/kg or 50 mg/kg per day while the rate of body weight reduction was within 10% even at the 18th day in the group receiving the administration of 12.5 mg/kg per day. Accordingly, in subsequent Examples, 6TG was decided to be repeatedly administered at 12.5 mg/kg once a day for 4 days.

(Example 4) Study of Dose of PT Using Nude Mouse

PT was repeatedly intraperitoneally administered to BALB/c nude mice (male, 7-week old, about 25 g in body weight, obtained from Japan SLC, Inc.) at 25, 50, 100, or 200 mg/kg once a day for 4 days. Body weight was measured at the 5th day from the first starting day of administration and later to determine the rate of body weight reduction. The rate of body weight reduction was calculated based on the following equation.

Body weight reduction rate (%) = [{(Body weight at day prior to the date of first PT administration) − (Body weight measured after start of PT administration)}/(Body weight at day prior to the date of first PT administration)] × 100

Saline was administered to a control group in place of PT. The results are shown in Table 3. In Table 3, they were expressed in average and standard deviation for 4 mice.

**Table 3**

| | | Elapsed Days from First Start of Administration | | | | |
|---|---|---|---|---|---|---|
| | | 5th Day | 9th Day | 12th Day | 16th Day | 18th Day |
| Rate of Body Weight Reduction (%) | Control Group | - 5.48±1.80 | -9.03±6.04 | -12.1±8.73 | -18.89±5.63 | -12.57±5.61 |
| | 25mg/kg/day | -8.42±12.36 | -12.11±13.3 | -10.94±12.04 | -14.11±11.25 | -15.38±9.65 |
| | 50mg/kg/day | -3.94±7.7 | -12.12±5.33 | -12.91±7.21 | -16.03±8.57 | -15.41±11.86 |
| | 100mg/kg/day | -5.03±8.95 | -12.82±9.29 | -16.86±8.46 | -20.08±9.11 | -21.05±6.18 |
| | 200 mg/kg/day | -5.75±9.93 | -15.25±10.49 | -18.1±9.64 | -18.1±9.64 | -17.19±9.86 |

As shown in Table 3, the rate of body weight reduction was within 10% even at the 18th day in all groups. Accordingly, in subsequent Examples, PT was decided to be repeatedly administered at 200 mg/kg once a day for 4 days.

(Example 5) Anti-tumor Effect of Administration of $^{64}$Cu-ATSM and 6TG in HT29 tumor-Bearing Mouse

The HT29 tumor-bearing model was prepared in the same way as Example 2, and 1 week after the implantation of HT29 cells, 6TG was repeatedly intraperitoneally administered at 12.5 mg/kg once a day for 4 days. At the final day of 6TG administration, 37 MBq (1 mCi) of $^{64}$Cu-ATSM was administered through the tail vein. Saline was administered to a control group in place of 6TG and $^{64}$Cu-ATSM. The tumor size in mice was measured 2, 4, 8, 12, or 15 days after starting the administration of 6TG.

(Comparative Example 4) Anti-tumor Effect of Administration of 6TG Alone in HT29 tumor-Bearing Mouse

The experiment was carried out in the same way as Example 5 except for administering saline in place of $^{64}$Cu-ATSM.

(Example 6) Anti-tumor Effect of Administration of $^{64}$Cu-ATSM and PT in HT29 tumor-Bearing Mouse

The HT29 tumor-bearing model was prepared in the same way as Example 2, and 1 week after the implantation of HT29 cells, PT was repeatedly intraperitoneally administered at 200 mg/kg once a day for 4 days. At the final day of PT administration, 37 MBq (1 mCi) of $^{64}$Cu-ATSM was administered through the tail vein. Saline was administered to a control group in place of PT and $^{64}$Cu-ATSM. The tumor size in mice was measured 2, 4, 8, 12, or 15 days after starting the administration of PT.

(Comparative Example 5) Anti-tumor Effect of Administration of PT Alone in HT29 tumor-Bearing Mouse

The experiment was carried out in the same way as Example 6 except for administering saline in place of $^{64}$Cu-ATSM.

[Evaluation 2]

The volume of tumor was calculated from the size of tumor measured in Examples 5 and 6 and Comparative Examples 4 and 5; the change thereof with time is shown in FIG. 4. In FIG. 4, the tumor volume at the day prior to the day of the start of 6TG or PT administration was set to 100%, and the percentage based thereon was given. In FIG. 4, data are expressed in average and standard deviation for 6 mice. In addition, the results of Example 2 and Comparative Examples 1 to 3 were shown together in FIG. 4. As shown in FIG. 4, the administration of 6TG or PT alone could not suppress the proliferation of tumor. The administration of $^{64}$Cu-ATSM alone could suppress the proliferation of tumor depending on the dose thereof, while the combined administration of 37 MBq of $^{64}$Cu-ATSM and 6TG or PT had a higher tumor proliferation-suppressing effect than the administration of 74 MBq of $^{64}$Cu-ATSM alone (Comparative Example 3).

(Example 7) Anti-tumor Effect of Repeated Administration of $^{64}$Cu-ATSM and 5-FU in HT29 tumor-Bearing Mouse

The HT29 tumor-bearing model was prepared in the same way as Example 2; 1 week after the implantation of HT29 cells, 5-FU was repeatedly intraperitoneally administered at 25 mg/kg once a day for 4 days; and at the final day (4th day) of 5-FU administration, 37 MBq (1 mCi) of $^{64}$Cu-ATSM was administered through the tail vein. The administration of 5-FU was again started at the 25th day from the start of 5-FU administration, with 25 mg/kg thereof repeatedly administered once a day for 4 days. At the final day of 5-FU administration, 37 MBq (1 mCi) of $^{64}$Cu-ATSM was then administered through the tail vein. Saline was administered to a control group in place of 5-FU and $^{64}$Cu-ATSM. The tumor size in mice was measured 2, 4, 8, 12, 14, 16, 20, 24, 27, 31, or 34 days after starting the administration of 5-FU.

(Comparative Example 6) Anti-tumor Effect of Administration of 5-FU Alone in HT29 tumor-Bearing Mouse

The experiment was carried out in the same way as Example 7 except for administering saline in place of $^{64}$Cu-ATSM.

(Comparative Example 7) Anti-tumor Effect of Administration of $^{64}$Cu-ATSM Alone in HT29 tumor-Bearing Mouse

The experiment was carried out in the same way as Example 7 except for administering saline in place of 5-FU.

[Evaluation 3]

The volume of tumor was calculated from the size of tumor measured in Example 7 and Comparative Examples 6 and 7; the change thereof with time is shown in FIG. 5. In FIG. 5, the tumor volume at the day prior to the day of the first start of 5-FU administration was set to 100%, and the percentage based thereon was given. In FIG. 5, data are expressed in average and standard deviation for 6 mice. As shown in FIG. 5, the administration of 5-FU alone could hardly suppress the proliferation of tumor. The administration of $^{64}$Cu-ATSM alone suppressed the proliferation of tumor depending on the dose thereof, while the combined administration of 37 MBq of $^{64}$Cu-ATSM and 5-FU had a higher tumor proliferation-suppressing effect than the administration of $^{64}$Cu-ATSM alone (Comparative Example 7). No body weight reduction was observed in all administration groups.

The above results showed that the combined use of $^{64}$Cu-ATSM and the metabolic inhibitor in an amount causing no body weight reduction was noted to have a significant anti-tumor effect without the observation of body weight reduction.

It is apparent that the present invention is not limited to the above embodiment, and may be modified and changed without departing from the scope and spirit of the invention.

An anti-tumor agent comprising a radioactive diacetyl-bis(N4-methylthiosemicarbazone) copper complex, wherein the anti-tumor agent is used for combined administration with a metabolic inhibitor.

## Claims

1. An anti-tumor agent comprising a radioactive diacetyl-bis(N4-methylthiosemicarbazone) copper complex, wherein the anti-tumor agent is used for combined administration with a metabolic inhibitor.

2. The anti-tumor agent according to claim 1, wherein the metabolic inhibitor is one or more agents selected from the group consisting of pyrimidine metabolism antagonists, purine metabolism antagonists, and folate metabolism antagonists.

3. The anti-tumor agent according to claim 1 or 2, wherein the metabolic inhibitor is a fluorinated pyrimidine agent.

4. The anti-tumor agent according to claim 3, wherein the fluorinated pyrimidine agent contains one or more compounds selected from the group consisting of 5-fluorouracil, tegafur, capecitabine, and salts thereof as an active ingredient.

5. The anti-tumor agent according to claim 1 or 2, wherein the metabolic inhibitor comprises 6-thioguanine or pemetrexed as an active ingredient.

6. The anti-tumor agent according to any one of claims 1 to 5, wherein the radioactive diacetyl-bis(N4-methylthiosemicarbazone) copper complex is $^{64}$Cu-ATSM or $^{67}$Cu-ATSM.

7. The anti-tumor agent according to any one of claims 1 to 6, wherein the anti-tumor agent is used as a cancer stem cell-killing agent.

8. The anti-tumor agent according to any one of claims 1 to 7, wherein the anti-tumor agent is used after the administration of the metabolic inhibitor.

9. The anti-tumor agent according to any one of claims 1 to 8, wherein the dose of the metabolic inhibitor is set so that when the metabolic inhibitor is administered alone, the rate of body weight reduction after the administration of the metabolic inhibitor is 10% or less.

10. Use of a metabolic inhibitor for combined administration with a radioactive diacetyl-bis(N4-methylthiosemicarbazone) copper complex.

11. Use of the metabolic inhibitor according to claim 10, wherein the metabolic inhibitor is used as a cancer stem cell-killing agent.

12. Use of the metabolic inhibitor according to claim 10 or 11, wherein the metabolic inhibitor comprises one or more compounds selected from the group consisting of 5-fluorouracil, tegafur, capecitabine, and salts thereof as an active ingredient.

**13.** An anti-tumor kit comprising:

a metabolic inhibitor and
a radioactive diacetyl-bis(N4-methylthiosemicarbazone) copper complex.

**14.** The anti-tumor kit according to claim 13, wherein the anti-tumor kit is used for killing cancer stem cells.

**15.** The anti-tumor kit according to claim 13 or 14, comprising a fluorinated pyrimidine agent as the metabolic inhibitor.

FIG. 1

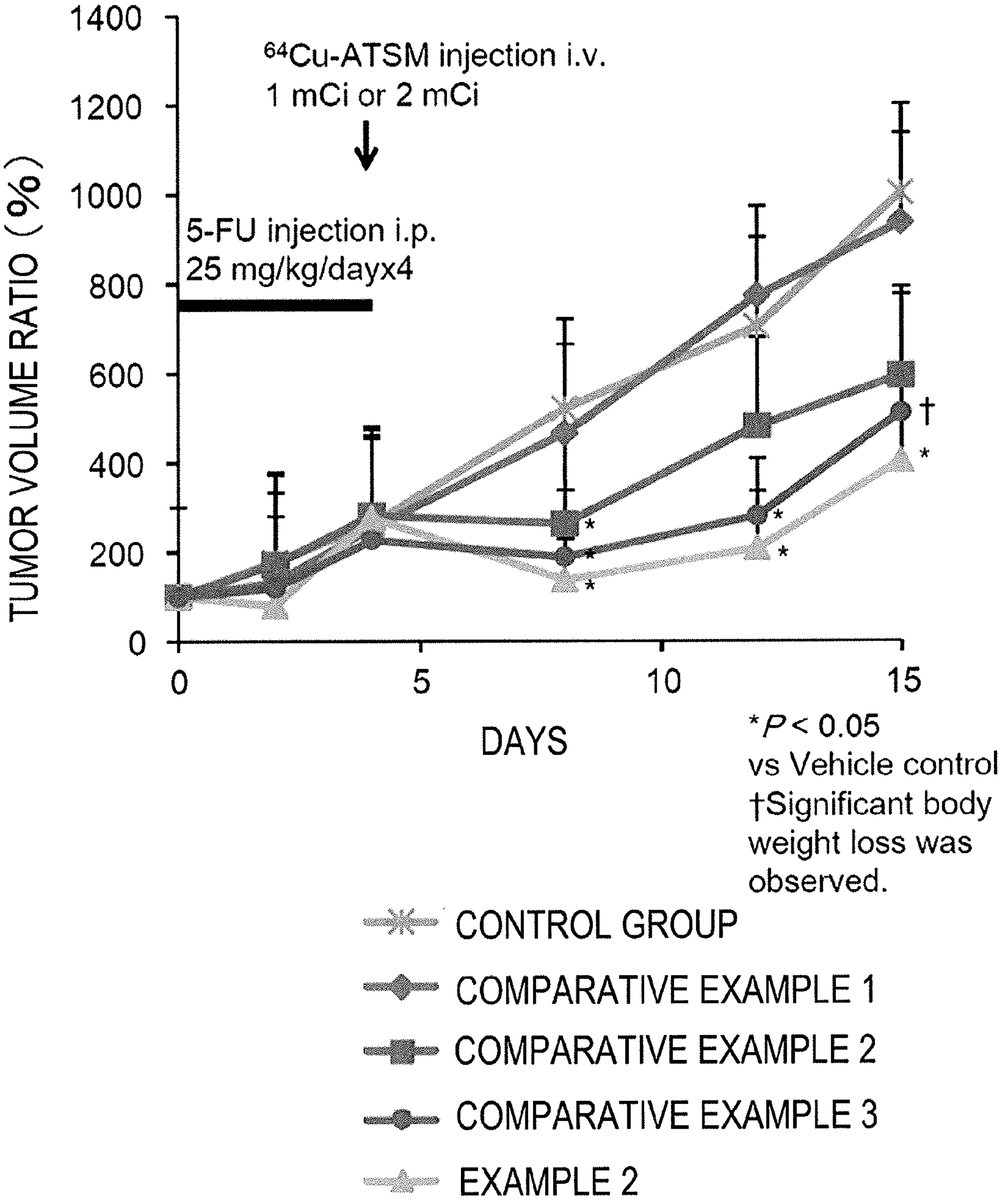
1400
1200
1000
800
600
400
200
0
TUMOR VOLUME RATIO (%)
64Cu-ATSM injection i.v.
1 mCi or 2 mCi
5-FU injection i.p.
25 mg/kg/dayx4
0
5
10
15
DAYS
*P< 0.05
vs Vehicle control
†Significant body weight loss was observed.
CONTROL GROUP
COMPARATIVE EXAMPLE 1
COMPARATIVE EXAMPLE 2
COMPARATIVE EXAMPLE 3
EXAMPLE 2

FIG. 2

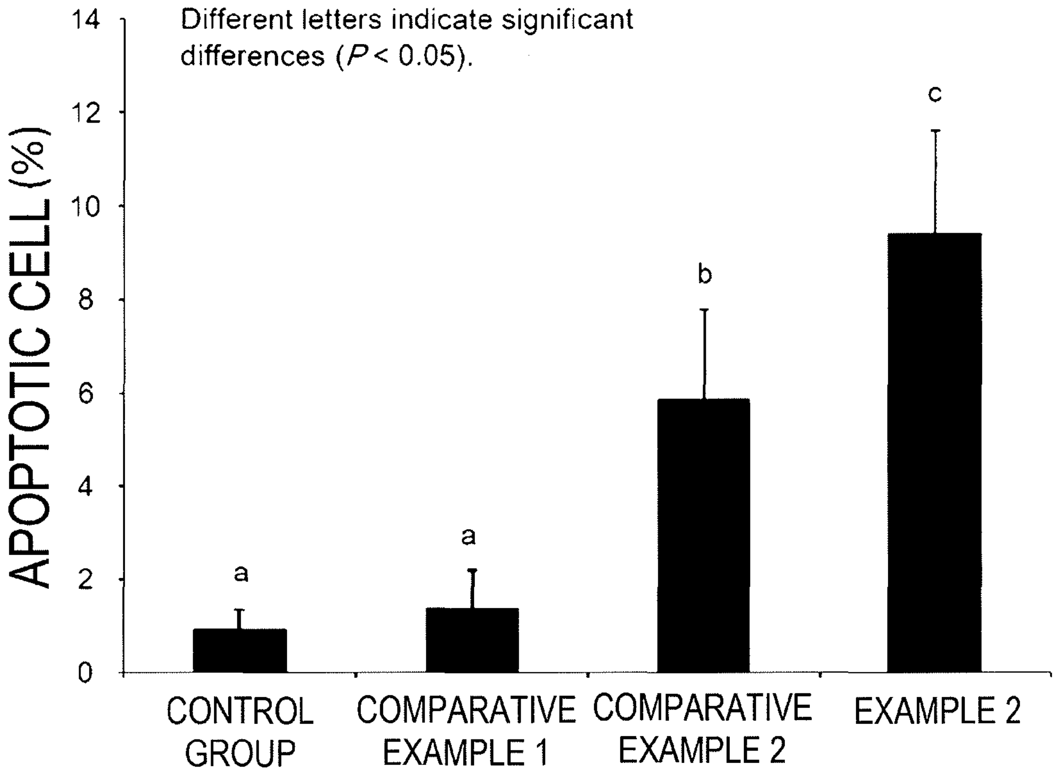
Different letters indicate significant differences (P< 0.05).
APOPTOTIC CELL (%)
14
12
10
8
6
4
2
0
a
a
b
c
CONTROL GROUP
COMPARATIVE EXAMPLE 1
COMPARATIVE EXAMPLE 2
EXAMPLE 2

FIG. 3

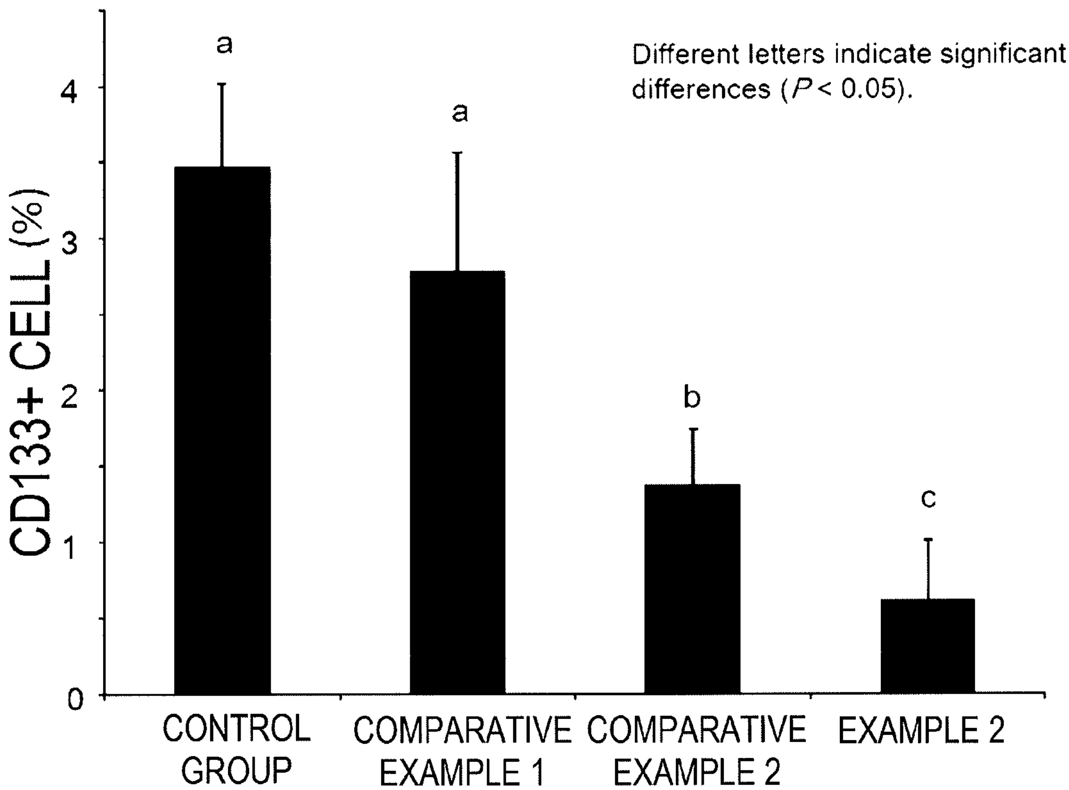
Different letters indicate significant differences ($P < 0.05$).
CD133+ CELL (%)
0
1
2
3
4
a
a
b
c
CONTROL GROUP
COMPARATIVE EXAMPLE 1
COMPARATIVE EXAMPLE 2
EXAMPLE 2

FIG. 4

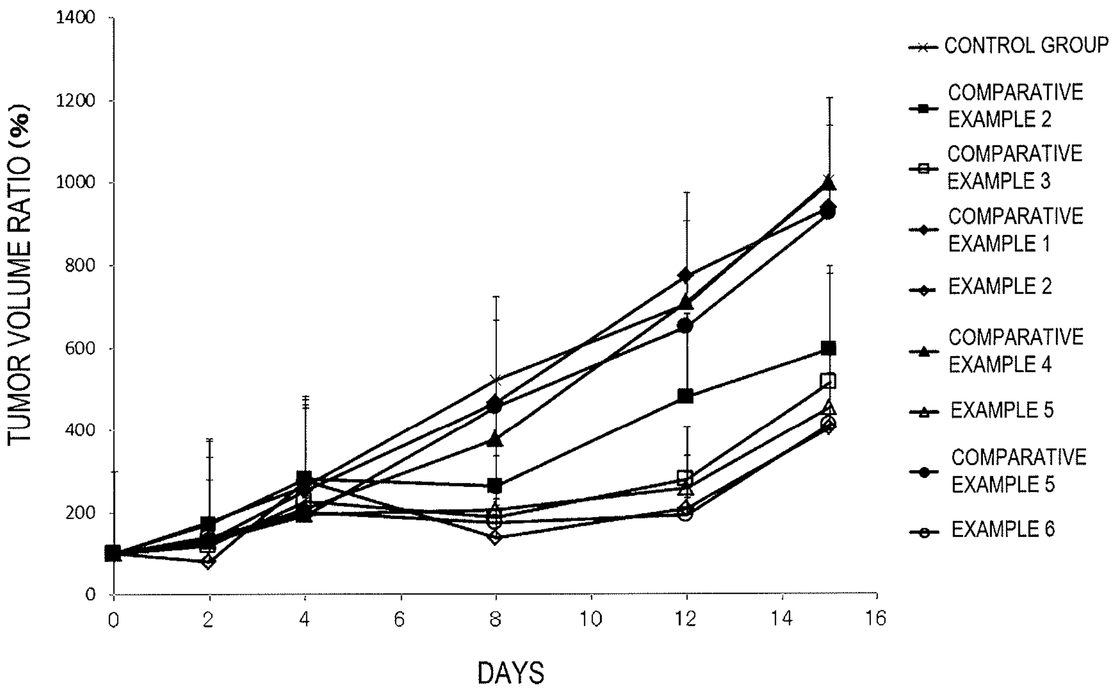
TUMOR VOLUME RATIO (%)
1400
1200
1000
800
600
400
200
0
0
2
4
6
8
10
12
14
16
DAYS
CONTROL GROUP
COMPARATIVE EXAMPLE 2
COMPARATIVE EXAMPLE 3
COMPARATIVE EXAMPLE 1
EXAMPLE 2
COMPARATIVE EXAMPLE 4
EXAMPLE 5
COMPARATIVE EXAMPLE 5
EXAMPLE 6

FIG. 5

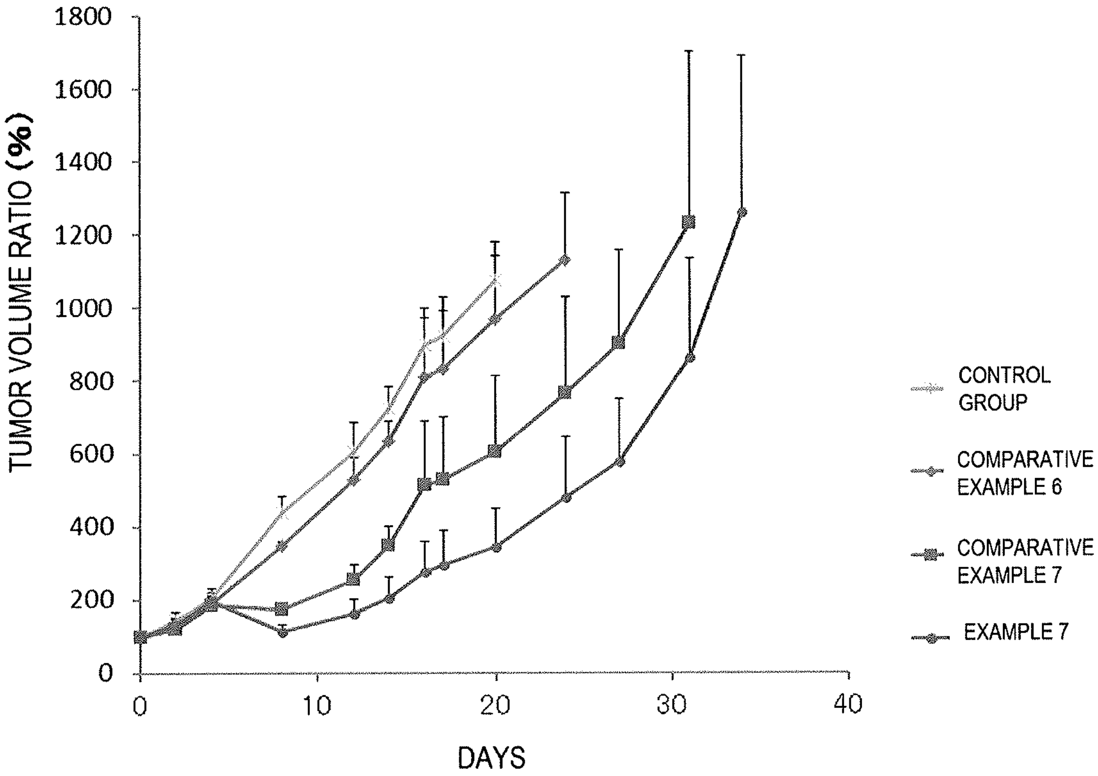
TUMOR VOLUME RATIO (%)
1800
1600
1400
1200
1000
800
600
400
200
0
0
10
20
30
40
DAYS
CONTROL GROUP
COMPARATIVE EXAMPLE 6
COMPARATIVE EXAMPLE 7
EXAMPLE 7

Europäisches Patentamt
European Patent Office
Office européen des brevets

# EUROPEAN SEARCH REPORT

Application Number
EP 13 19 9364

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>Y | JP 2010 013380 A (UNIV FUKUI)<br>21 January 2010 (2010-01-21)<br>* paragraphs [0001], [0029] * | 1-15<br>2,3,15 | INV.<br>A61K51/04<br>A61K31/555<br>A61K31/513<br>A61K31/519<br>A61K31/52<br>A61P35/00 |
| X | DAVID W DIETZ ET AL: "Tumor Hypoxia Detected by Positron Emission Tomography with 60Cu-ATSM as a Predictor of Response and Survival in Patients Undergoing Neoadjuvant Chemoradiotherapy for Rectal Carcinoma: A Pilot Study",<br>DISEASES OF THE COLON & RECTUM, SPRINGER-VERLAG, NE,<br>vol. 51, no. 11,<br>6 August 2008 (2008-08-06), pages 1641-1648, XP019638127,<br>ISSN: 1530-0358, DOI: 10.1007/S10350-008-9420-3<br>* page 1641, "methods" * | 1-5, 7-10, 12-15 | |
| X | REBECCA L AFT ET AL: "Enhancing Targeted Radiotherapy by Copper(II)diacetyl-bis(N4-methylthiosemicarbazone) Using 2-Deoxy-d-Glucose 4 N",<br>CANCER RESEARCG,<br>vol. 63, 1 September 2003 (2003-09-01), pages 5496-5504, XP055109527,<br>* whole document and especially: the abstract; page 5497, "animal models" * | 1-10,13, 14 | TECHNICAL FIELDS SEARCHED (IPC)<br>A61K |
| Y | "THE MERCK INDEX, AN ENCYCLOPEDIA OF CHEMICALS, DRUGS, AND BIOLOGICALS, 14th Edition",<br>1 January 2006 (2006-01-01), MERCK & CO., INC., WHITEHOUSE STATION, NJ, USA,<br>XP055110364,<br>page THER-13,<br>* page THER-13, "antimetabolites" * | 2,3,15 | |

The present search report has been drawn up for all claims

1

| Place of search | Date of completion of the search | Examiner |
|---|---|---|
| The Hague | 31 March 2014 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 19 9364

This annex lists the patent family members relating to the patent documents cited in the above-mentioned European search report. The members are as contained in the European Patent Office EDP file on
The European Patent Office is in no way liable for these particulars which are merely given for the purpose of information.

31-03-2014

| Patent document cited in search report | Publication date | Patent family member(s) | Publication date |
|---|---|---|---|
| JP 2010013380 A | 21-01-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012289452 A
- JP 2013250743 A
- JP H08245425 B
- JP 2010013380 A


### Non-patent literature cited in the description


- **JASON S. LEWIS et al.** *Pros. Natl. Acad. Sci.,* 2001, vol. 98, 1206-1211
- **YUKIE YOSHII et al.** *Nucl. Med. Biol.,* 2010, vol. 37, 395-404
- **YUKIE YOSHII et al.** *Nucl. Med. Biol.,* 2011, vol. 38, 151-157
- **JALILIAN et al.** *Acta Pharmaceutica,* 2009, vol. 59 (1), 45-55
- PET yo Houshaseiyakuzai no Seizo oyobi Hinshitsukanri - Gousei to Rinshoushiyou eno Tebiki. 2011
- **TANAKA et al.** *Nuclear Medicine and Biology,* 2006, vol. 33, 743-50
- **LEWIS et al.** *J. Nucl. Med.,* 2001, vol. 42, 655-661
- **MCCARTHY et al.** *Nuclear medicine and biology,* 1997, vol. 24, 35-43
- **OBATA et al.** *Nuclear medicine and biology,* 2003, vol. 30, 535-539